# EUROPEAN PATENT APPLICATION

(11) **EP 4 489 018 A1**
(43) Date of publication of application: **08.01.2025**
(21) Application number: 24186218.4
(22) Date of filing: 03.07.2024
(51) Int. Cl.: G16H 20/40, G16H 50/20, G16H 50/70, A61B 5/361, G06N 3/045

(54) **ADVANCED ATRIAL FIBRILLATION TREATMENT SYSTEM AND METHOD UTILIZING CROWDSOURCED MACHINE LEARNING MODELS**

(30) Priority: 03.07.2023 US 202363524712 P; 18.12.2023 US 202363611303 P
(71) Applicant: Biosense Webster (Israel) Ltd., 2066717 Yokneam (IL)
(72) Inventor: AMOS, Yariv Avraham, 2066717 Yokneam (IL); ELIYAHU, Shiran, 2066717 Yokneam (IL); TSOREF, Liat, 2066717 Yokneam (IL); SHALGI, Avi, 2066717 Yokneam (IL)
(74) Representative: Carpmaels & Ransford LLP

(57) **Abstract**

The presently disclosed subject matter includes computer methods and computer systems that enable to select and provide a suitable treatment for AF patients that increases the likelihood of long-term amelioration of AF conditions, and thereby enhances treatment of AF patients. The disclosure provides methods and systems for analysis of the condition of AF patients and the tailoring of personalized treatment regimens based on various AF features obtained from the patients, and the determination of a treatment selected from at least PVI only and PVI plus. To overcome technical difficulties resulting from scarcity of data a machine learning model that is trained using physician recommendation rather than observed treatment outcome is disclosed.

## Description

### CROSS-REFERENCE TO RELATED APPLICATION

This application claims the benefit of U.S. Provisional Patent Application No. 63/524,712, filed July 3, 2023, and U.S. Provisional Application No. 63/611,303 filed December 18, 2023, which are incorporated herein by reference.

### TECHNICAL FIELD

The presently disclosed subject matter relates to computer systems and computer implemented methods for the treatment of atrial fibrillation.

### BACKGROUND

Atrial fibrillation (AF) is the most common diagnosed sustained arrhythmia and is characterized by rapid and irregular activation of the atria. Atrial fibrillation can be paroxysmal, lasting 7 days or less with or without intervention, or may be continuous beyond 7 days (persistent, PS-AF) or beyond 12 months (long-standing, LS-PS-AF). Permanent AF is the term used for longstanding persistent AF when any attempt to restore sinus rhythm has been abandoned or has proved impossible. See e.g., Kaba, R. A., Momin, A. & Camm, J. Persistent atrial fibrillation: The role of left atrial posterior wall isolation and ablation strategies. J. Clin. Med. 10, (2021).

Treatment strategies vary, depending on the extent and duration of AF. Pulmonary vein isolation (PVI) is a medical procedure that aims to restore normal heart rhythm and reduce or eliminate the symptoms associated with AF. However, in severe cases of AF, PVI alone may not be sufficient, and long-term failure of a "PVI only" is observed.

### GENERAL DESCRIPTION

Evidence suggests a consistent link between the enlargement of the left atrium volume and the aggravation of both the severity and chronic nature of atrial fibrillation (AF) in affected patients. The shape of the atrium has been known to change as well, turning from a tube-like shape to a sphere shape as the disease progresses.

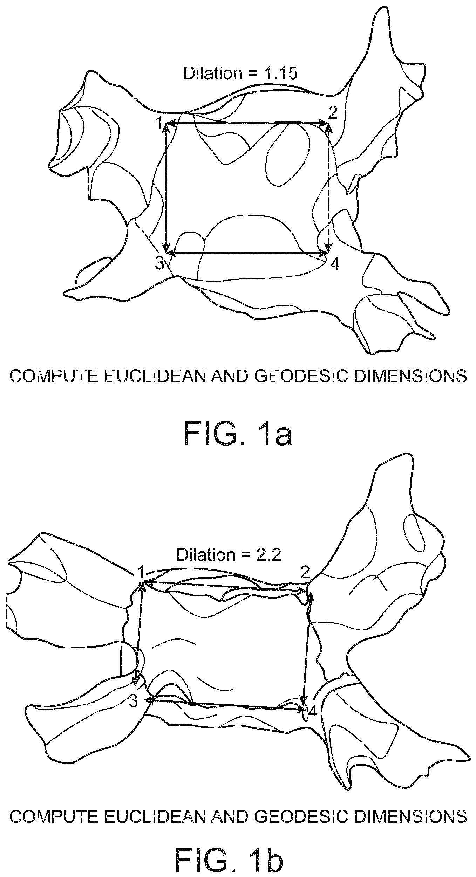

Figs. 1a and 1b show imaging system output of the left atrium of two AF patients. In this example, the dilation values (in the current case, Euclidean dilation values) are calculated by dividing the width of the posterior wall by its length.

The methods and systems disclosed herein enable to select and provide a suitable treatment for AF patients, which increases the likelihood of long-term amelioration of AF conditions, and thereby enhances treatment of AF patients.

It is disclosed herein that changes (e.g., dilations) in different sub-compartments (or "chambers") of the heart, observed and measured in specific anatomical parts of different sub-compartments, can be used to predict long-term success or failure of PVI only treatment and/or PVI plus treatment in AF patients.

Considering the left atrium as an example, it has been found by the inventors that geometrical transformation, such as dilation, in each one of its anatomical parts, including posterior, anterior, left lateral, right lateral, roof and inferior walls, has a predictive quality with respect to the outcome of PVI only treatment. According to findings of the inventors, certain geometrical properties (or specific combinations of geometrical properties) of the left atrium, observed in AF patients, are indicative of whether a long-term success or long-term failure is expected following a PVI only treatment, and accordingly assist in selecting an appropriate treatment to be administered to the patients, selected between PVI only and PVI plus.

According to some examples, an index (and a novel technique utilizing the index) is proposed that is indicative of the likelihood of long-term success of the PVI "only" procedure in atrial fibrillation subjects. The index can be used to select patients for one of PVI only or different PVI plus. The index is generated based on heart related features including the geometric properties and possibly other heart related features, such as voltage related features and cycle length. Additional details of a statistical analysis, performed on data obtained from AF patients that supports the above principles, is provided below in the "examples" section.

One challenge in using geometrical properties of sub-compartments of the heart is related to the fact that the imaging output of heart imaging systems is often impaired or incomplete and includes unmapped regions. This shortcoming creates difficulties, and, in some cases, precludes the ability to determine changes in the overall volume and/or shape of the heart and/or atria, thus creating a technical setback in analysis of the condition of the heart in AF patients and in selecting a proper treatment.

The subject matter disclosed herein includes a new approach for predicting success of a PVI treatment, which is effective also in cases of incomplete imaging output. According to this approach, a collection of anatomical prediction parameters (otherwise referred to as "success prediction parameters") can be used, each parameter being calculated based on geometrical data collected from a respective anatomical part of the heart. The multiplicity and variety of parameters, and the fact that different anatomical prediction parameters are calculated based on different respective anatomical parts, provide redundancy in the data, such that even if the imaging output of the heart is incomplete, the mapped areas of the heart provide sufficient output that can be used for calculating the parameters and providing the desired prediction based on these parameters.

The subject matter disclosed herein further includes a computer system and method dedicated for recommending/selecting an appropriate ablation approach for AF patients using a machine learning (ML) model. A machine learning classifier is trained, using a training dataset that includes multiple features determined with respect to different patients in an AF patients-database (herein below "AF related features"), to correlate between various combinations of AF related features and a respective probability of success of PVI only procedure and/or PVI plus procedure.

According to one approach, the training data includes information indicating whether a PVI only or a PVI plus treatment was administered to each patient, and the observed long-term success of the AF treatment. AF related features are labeled according to the observed success or failure of the treatment, thus enabling the ML model to associate between AF related features and a recommended AF treatment.

According to another example, the training data includes information indicating the PVI treatment (ablation approach), which was recommended by physicians treating the patients, rather than the actual observed outcome of the treatment. As further explained below, this approach is based on the wisdom of the crowd (WoC) principle, enabling the ML model to associate between AF related features and a treatment of choice selected by physicians. This assists in overcoming the technical challenge resulting from lack of sufficient data on the observed outcome of AF treatments.

The present disclosure also relates to the field of personalized medicine. More specifically, the disclosure provides methods and systems for analysis the condition of an AF patient and the tailoring of personalized treatment, selected from at least PVI only and PVI plus, based on various AF features obtained from the patient.

### SUMMARY

According to a first aspect of the presently disclosed subject matter there is provided a computer-implemented method of selecting an Atrial Fibrillation (AF) treatment to AF patients, the method comprising:
obtaining a plurality of AF related features of a patient; the AF related features of the patient include heart-related features characterizing various heart-related attributes of the patient;
the heart-related features include at least one of: one or more anatomical prediction parameters, each anatomical prediction parameter being calculated based on geometrical data collected from a particular anatomical part of the heart; and one or more voltage-related features;
applying a machine learning (ML) model to the plurality of AF related features of the patient; wherein the ML model has been trained using a training dataset that includes multiple data points, each data point corresponding to an AF patient associated with AF related features and a corresponding recommendation made by a physician to administer to the patient an AF treatment selected from a group including at least Pulmonary Vein Isolation (PVI) and PVI plus treatments, thus training the ML model to provide, during inference, data indicating a recommended AF treatment to a given patient according to the AF related features of the given patient;
generating an ML model output indicative of a recommended AF treatment for the patient, selected from a Pulmonary Vein Isolation (PVI) only procedure, and a PVI plus procedure; and
providing data indicative of the recommended AF treatment to a healthcare provider.

In addition to the above features, the method according to this aspect of the presently disclosed subject matter can optionally comprise one or more of features i to xi listed below, in any technically possible combination or permutation:
i. Wherein obtaining the AF related features includes performing one or more procedures on the patient.
ii. wherein the heart related features include anatomical prediction parameters, the method further comprising obtaining anatomical prediction parameters of the patient:
   performing a procedure on the patient for obtaining a 3D anatomical map of at least one sub-compartment of a heart of the patient, selected from a group comprising: left atrium, right atrium, and left ventricle;
   segmenting the anatomical (3D) map to thereby obtain multiple anatomical parts of the at least one sub-compartment;
   for at least part of the multiple anatomical parts, determining at least one respective dimension;
   determining anatomical prediction parameters, wherein each anatomical prediction parameter is determined based on one or more dimensions of an anatomical part of the at least one sub-compartment of the heart of the patient.
iii. The method further comprises determining at least one anatomical prediction parameter by applying a mathematical operation between different dimensions of a respective anatomical part of the at least one sub-compartment.
iv. Wherein the AF related features further include one or more of: cycle length, medical history, and patients' demographics.
v. Wherein the ML model is an ensemble of classifiers, each classifier being trained on a subset of the data points and configured to provide a respective output indicative of a recommended AF treatment for the patient; and wherein a final recommendation is determined based on a majority vote.
vi. Wherein each subset of data points includes patients that were recommended by a particular physician, lab, or medical institution, thus representing a virtual physician.
vii. Wherein the machine learning model output is further indicative of a respective significance of different AF related features in determining the recommended AF treatment for the patient.
viii. Wherein, during training, clustering is applied on the multiple data points to thereby assign a subset of patients characterized by similar AF related features to respective groups, each group representing a patient type, and tagging the patients in the group according to distribution of recommendations made by physicians in each group.
ix. Wherein the ML output, that includes a recommendation to apply a PVI plus procedure on the patient, also includes information indicative of a particular type of recommended PVI plus procedure.
x. The method comprises determining the anatomical prediction parameters by applying a mathematical operation between different dimensions of a respective anatomical part.
xi. The method comprising performing one or more procedures on the patient to obtain the heart-related features from the patient.

The presently disclosed subject matter further contemplates a computer system comprising at least one processing circuitry configured to perform the method of selecting an Atrial Fibrillation (AF) treatment to AF patient according to the first aspect above.

The presently disclosed subject matter further contemplates a non-transitory program storage device readable by a computer, tangibly embodying a program of instructions executable by the computer to perform the methods disclosed according to the first aspect above.

The presently disclosed subject matter further contemplates a computer implemented method and a computer system (and a respective a non-transitory computer storage device) for inducing long-term amelioration to an Atrial Fibrillation (AF) patient, the method comprising performing the operations as set forth by the first aspect above and administering to the patient PVI only treatment or PVI plus as prescribed by the recommendation.

The presently disclosed subject matter further contemplates a computer implemented method and a computer system (and a respective a non-transitory computer storage device) for predicting a response to a Pulmonary Vein Isolation (PVI) treatment administered to an Atrial Fibrillation (AF) patient, the method comprising operations according to the first aspect above.

The presently disclosed subject matter further comprises a computer-implemented method and system (and a respective a non-transitory computer storage device) dedicated to train a machine learning (ML) model dedicated for selecting/recommending treatment to AF (Atrial Fibrillation) patients by providing data indicating a recommended treatment selected between Pulmonary Vein Isolation (PVI) only and PVI plus treatments; the method comprising:
generating a training dataset comprising multiple data points, each data point corresponding to an AF patient associated with AF related features and a corresponding recommendation made by a physician to administer to the patient an AF treatment selected from a group including at least Pulmonary Vein Isolation (PVI) and PVI plus treatments;
training the ML model using the generated dataset, to associate specific AF related features with corresponding physician recommendations for AF treatment, thereby enabling the ML model to provide, during inference, data indicating a recommended AF treatment for a given patient based on the AF related features of the given patient; wherein the use of physician recommendations in training the ML model addresses the technical problem of data scarcity related to the actual outcomes of AF treatments.

According to another aspect of the presently disclosed subject matter there is provided a computer program product stored on a (non-transitory) computer storage device, the computer program product comprising software executable by a computer, the software including a machine learning (ML) model for recommending AF (Atrial Fibrillation) treatments to AF patients, wherein the software is produced by a process comprising:
generating a training dataset comprising multiple data points, each data point corresponding to an AF patient associated with AF related features and a corresponding recommendation made by a physician to administer to the patient an AF treatment selected from a group including at least Pulmonary Vein Isolation (PVI) and PVI plus treatments; training the ML model using the generated dataset to associate specific AF related features with corresponding physician recommendations for AF treatment, thereby enabling the ML model to provide, during inference, data indicating a recommended AF treatment for a given patient based on the AF related features of the given patient.

According to further aspects of the presently disclosed subject matter there is provided a computer system and a computer-implemented method (and a respective a non-transitory computer storage device) for determining a personalized treatment for an AF patient, the method comprising:
obtaining a plurality of AF related features of a patient; the AF related features of the patient include heart-related features characterizing various heart-related attributes of the patient;
the heart-related features include at least one of: one or more anatomical prediction parameters, each anatomical prediction parameter being calculated based on geometrical data collected from a particular anatomical part of the heart; and one or more voltage-related features;
applying a machine learning (ML) model to the plurality of AF related features of the patient; wherein the ML model has been trained using a training dataset that includes multiple data points, each data point corresponding to an AF patient associated with AF related features and a corresponding recommendation made by a physician to administer to the patient an AF treatment selected from a group including at least Pulmonary Vein Isolation (PVI) and PVI plus treatments, thus training the ML model to provide, during inference, data indicating a recommended AF treatment to a given patient according to the AF related features of the given patient;
generating an ML model output indicative of a recommended AF treatment for the patient, selected from a Pulmonary Vein Isolation (PVI) only procedure, and a PVI plus procedure; and
providing data indicative of the recommended AF treatment to a healthcare provider.

Systems, methods, non-transitory program storage devices, and computer program products disclosed above in accordance with various aspects of the presently disclosed subject matter can optionally comprise one or more of features (i) to (xi) listed above, *mutatis mutandis,* in any technically relevant combination or permutation.

### BRIEF DESCRIPTION OF THE DRAWINGS

In order to understand the invention and to see how it can be carried out in practice, embodiments will be described, by way of non-limiting examples, with reference to the accompanying drawings, in which:
Figs. **1a** and **1b** are illustrations of a dilated left atrium in AF patients;
Fig. 2 is a block diagram of a computer system for enhanced treatment of AF patients in accordance with some examples of the presently disclosed subject matter;
Fig. 3a is a flowchart showing operations carried out as part of enhanced recommendation and treatment of AF patients, in accordance with some examples of the presently disclosed subject matter;
Fig. **3b** is a flowchart showing operations carried out as part of machine learning based enhanced recommendation and treatment of AF patients, in accordance with some examples of the presently disclosed subject matter;
Fig. 4 shows various stages of the computer analysis in accordance with some examples of the presently disclosed subject matter;
Fig. 5 is an image showing four anchor points of the posterior wall, in accordance with some examples of the presently disclosed subject matter;
Fig. 6 is a flowchart showing operations carried out for training multiple machine learning models according to the WoC approach, in accordance with some examples of the presently disclosed subject matter;
Fig. 7 schematically illustrates the inference using an ensemble of classifiers, in accordance with some examples of the presently disclosed subject matter;
Fig. 8 is a block diagram of a computer system that combines the functionality of system **200** with additional functionalities related to identification of ablation lines in accordance with some examples of the presently disclosed subject matter;
Fig. 9 is a graph showing a specific example of correlation between right lateral wall or septum (RLAT) width parameters and long-term success of PVI only treatment, in accordance with some examples of the presently disclosed subject matter; and
Fig. **10** presents a graph illustrating the survival probability of AF patients over time, in accordance with some examples of the presently disclosed subject matter.

### DETAILED DESCRIPTION

In the following detailed description, numerous specific details are set forth in order to provide a thorough understanding of the invention. However, it will be understood by those skilled in the art that the presently disclosed subject matter may be practiced without these specific details. In other instances, well-known methods, procedures, components, and circuits have not been described in detail so as not to obscure the presently disclosed subject matter.

Unless specifically stated otherwise, as apparent from the following discussions, it is appreciated that throughout the specification discussions utilizing terms such as "obtaining", "applying", "generating", "providing", or the like, refer to the action(s) and/or process(es) of a computer that manipulate and/or transform data into other data, said data represented as physical, such as electronic, quantities and/or said data representing the physical objects.

The terms "computer", "computer system", "computer device", "computerized device", or the like, should be expansively construed to include any kind of hardware-based electronic device with one or more data processing circuitries. Each processing circuitry can comprise, for example, one or more processors operatively connected to computer memory, loaded with executable instructions for executing operations, as further described below.

The one or more processors referred to herein can represent, for example, one or more general-purpose processing devices, such as a microprocessor, a central processing unit, or the like. More particularly, a given processor may be one of: a complex instruction set computing (CISC) microprocessor, a reduced instruction set computing (RISC) microprocessor, a very long instruction word (VLIW) microprocessor, a processor implementing other instruction sets, or a processor implementing a combination of instruction sets. The one or more processors may also be one or more special-purpose processing devices such as an application specific integrated circuit (ASIC), a field programmable gate array (FPGA), a digital signal processor (DSP), a graphics processing unit (GPU), a network processor, or the like.

The memories referred to herein can comprise for example, one or more of the following: internal memory, such as, e.g., processor registers and cache, etc., main memory such as, e.g., read-only memory (ROM), flash memory, dynamic random-access memory (DRAM) such as synchronous DRAM (SDRAM) or Rambus DRAM (RDRAM), etc.

Some of the operations in accordance with the teachings herein may be performed by a computer device specially constructed for the desired purposes, or by a general-purpose computer specially configured for the desired purpose by a computer program stored in a computer readable storage medium.

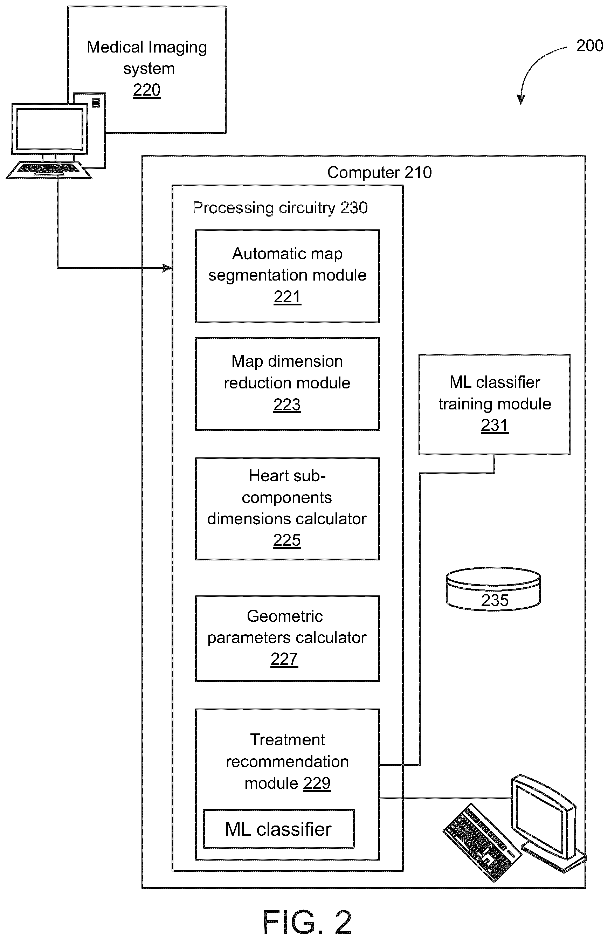

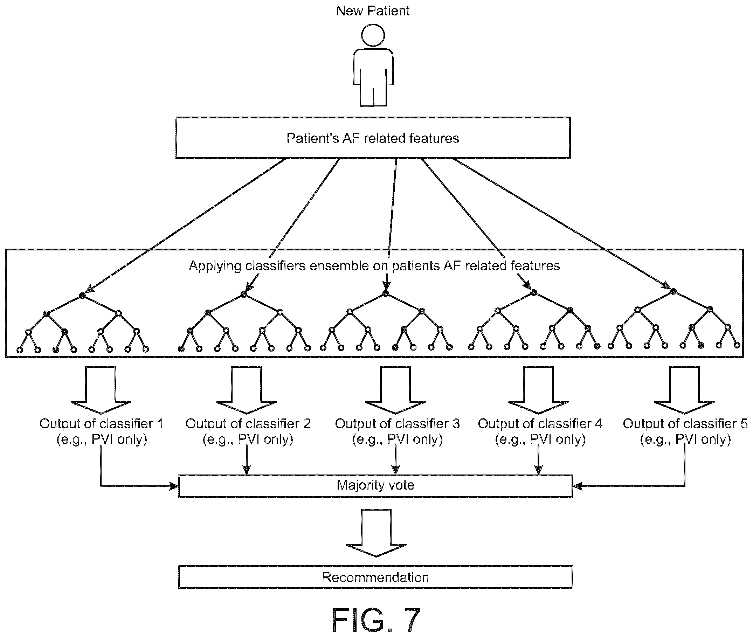

Figs. 2 and 7 illustrate a general schematic block diagram of a system in accordance with some examples of the presently disclosed subject matter. Different components in Figs. 2 and 7 can be made up of any combination of software, hardware, and/or firmware that performs the functions as defined and explained herein. The components in Figs. 2 and 7 may be centralized in one location and/or on one device, or dispersed over more than one location or device. For example, while Figs. 2 and 7 show computer system **220** and computer **210** as two separate computers, in other examples the two computer systems can be integrated in one computer system. In different examples of the presently disclosed subject matter, the system may comprise fewer, more, and/or different modules than those shown in Figs. 2 and 7.

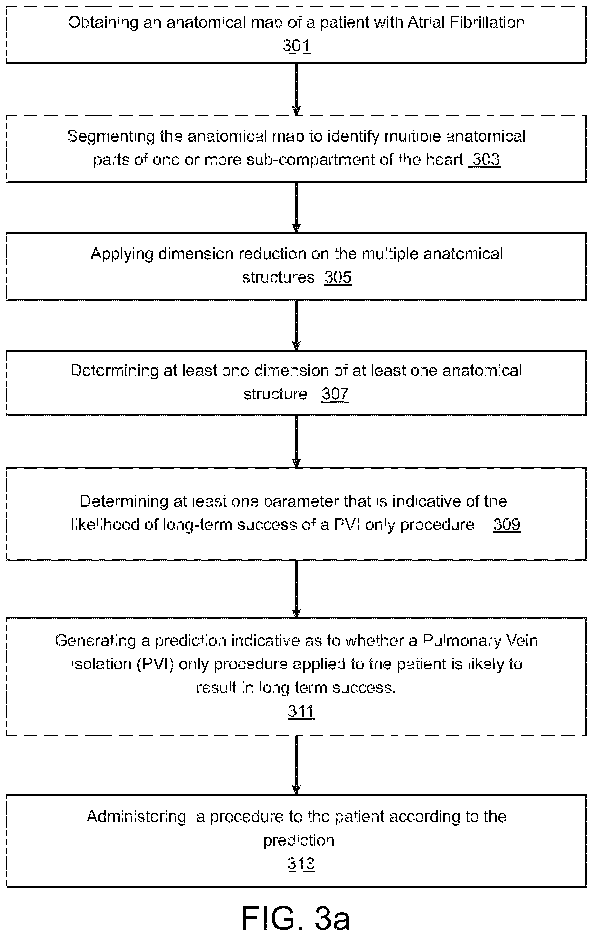

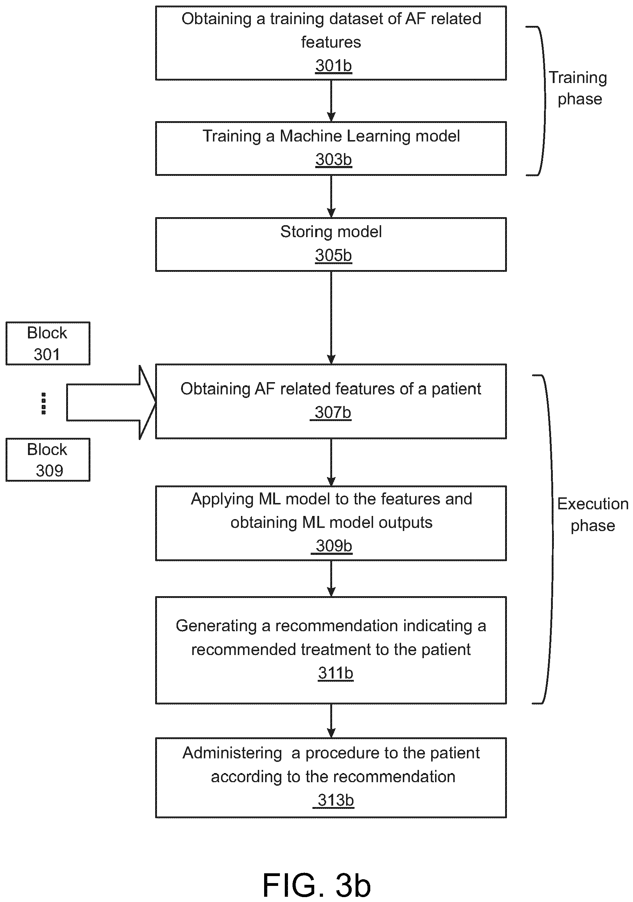

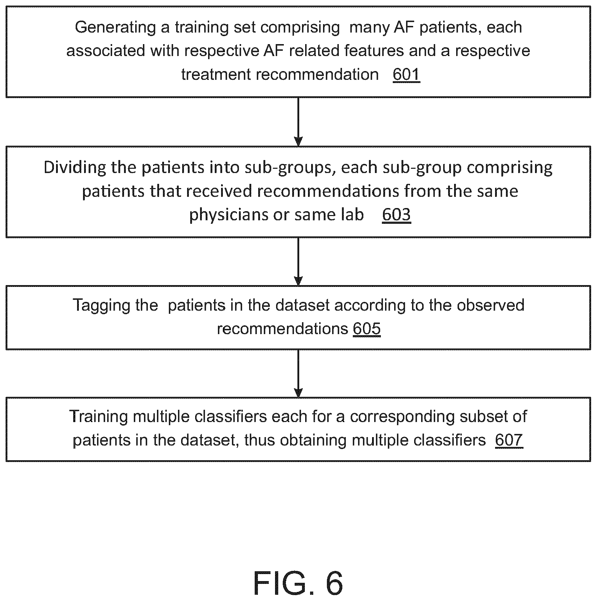

Figs. 3a, **3b** and 6 are flowcharts illustrating operations carried out in accordance with examples of the presently disclosed subject matter. According to different examples of the presently disclosed subject matter, fewer, more and/or different stages than those shown in Figs. 3a, 3b, and 6 may be executed. According to different examples of the presently disclosed subject matter, one or more stages illustrated in Figs. 3a, 3b, and 6 may be executed in a different order and/or one or more groups of stages may be executed simultaneously for multiple commands.

It is noted that any reference made in the description to the left atrium, is done by way of non-limiting example only, and the presently disclosed subject matter also contemplates applying the same principles in a similar manner to other sub-compartments of the heart, including for example, the right atrium, the left ventricle, right ventricle, and blood vessels connected to the heart, such the pulmonary veins.

Reference is now made to Fig. 2, showing a schematic block diagram of a computer system according to some examples of the presently disclosed subject matter. System **200** comprises computer **210** (also referred to as "AF treatment recommendation and treatment computer system") configured inter alia, to analyze an anatomical map of the heart of an AF patient and provide a recommendation, with respect to a preferred method of PVI treatment. Computer system **210** illustrated in Fig. 2 can comprise at least one processing circuitry **230** comprising one or more computer processors configured to execute several functional modules in accordance with computer-readable instructions implemented on a non-transitory computer-readable storage medium. Such functional modules are referred to hereinafter as comprised in the processing circuitry 230. Notably, while system 210 shows a single processing circuitry, it can otherwise include more than one processing circuitry, e.g., with different circuitries comprising different functional modules.

An anatomical map (or model) of the heart can be generated by computer 210 or received from another computer system. The anatomical map of the heart can be generated using an anatomical image of the heart received from a suitable medical imaging (computer) system (220). Generation of the anatomical map may involve, for example, using a fast anatomical mapping (FAM) technique available in the CARTOTM system, produced by Biosense Webster Inc. (Irvine, Calif.), or using any other suitable technique, or using any suitable combination of the above. The map of the heart may comprise any suitable type of three-dimensional (3D) anatomical map produced using any suitable technique. The map may come from an intracardial ECG (electrocardiogram) and may comprise both location information, of a catheter moving around the heart sub-componenets, and ECG measurements, such as voltage at each location.

In some examples, processing circuitry 230 comprises automatic map segmentation module 221 configured to automatically segment the 3D map of the heart into its various sub-compartments, and further segment one or more sub-compartments into the respective anatomical parts, such as posterior and anterior walls, left lateral, roof wall, inferior wall, septum, mitral, left atrial appendage (LAA), and 4 pulmonary veins (LSPV, LIPV, RSPV, RIPV). Notably, while all the anatomical parts listed above may be provided during segmentation, not all of them are necessarily considered during the analysis and treatment recommendation process.

Processing circuitry 230 may further include map dimensions reduction module 223 configured to transform the 3D map into a 2D representation of the map; heart sub-compartment dimensions calculator 225 configured to measure or calculate the dimensions of various sub-compartments of the heart, e.g., various anatomical parts of the left atrium; geometric parameters calculator 227 configured to calculate at least one parameter (also referred to herein as "anatomical prediction parameter"); and treatment recommendation module 229 configured to determine and provide a recommendation as to whether or not PVI only treatment is likely to be successful if administered to the patient. Computer system 210 may further comprise a computer interface that includes, for example, keyboard and mouse and computer display, where the data can be displayed e.g., to a healthcare provider, using an appropriate graphical user interface program.

Turning to Fig. 3a, this is a flowchart showing operations carried out as part of an analysis and treatment recommendation process in accordance with some examples of the presently disclosed subject matter. For ease of understanding and by way of non-limiting example only, the description of operations disclosed in Fig. 3a is made with reference to the components shown in system 200.

The process described hereinbelow can be applied for analyzing the heart of a patient suffering from AF, and determining whether a PVI only procedure is likely to be the right choice of treatment for the patient, e.g., is likely to provide long-term success if administered to the patient. According to one example, the term "long-term success" is meant to include a period without AF as well as any arrhythmia ("AF and arrhythmia-free period") following treatment, lasting between 3 to 18 months. According to one specific example, long-term success refers to AF and an arrhythmia-free period of at least 3 months following treatment. According to another specific example, long-term success refers to AF and an arrhythmia-free period of at least 6 months following treatment. According to another specific example, long-term success refers to AF and an arrhythmia-free period of at least 9 months following treatment. According to another specific example, long-term success refers to AF and an arrhythmia-free period of at least 12 months following treatment. According to another specific example, long-term success refers to AF and an arrhythmia-free period of at least 15 months following treatment. According to another specific example, long-term success refers to AF and an arrhythmia-free period of at least 18 months following treatment.

The terms "improving", "augmenting" or "enhancing" the AF treatment refer to the determination of an AF treatment that would result in a longer AF and arrhythmia-free period, as compared to the alternative AF treatment.

The term PVI only (or "PVI alone") refers to an ablation procedure which is applied only on the pulmonary veins of the left atrium, where PVI plus refers to additional ablations applied to additional anatomical parts of the heart. Additional parts targeted in PVI plus include for example one or more of:
RoofLine; LeftCarina; RightCarina; PosteriorLine; InferiorLine; MitralLine; AnteriorLine; LAAlsolation; CavoTricuspidlsthmus; RAAlsolation; InferiorVenaCavalsolation; SuperiorVenaCavalsolation; Substrate Modification; Substrate Isolation; Fractionation; Focal; Rotor; LA_PosteriorWall; CoronarySinus; RA_FreeWall; LA_AnteriorWall; LA_LateralWall; LA_lnferiorWall;RA_Septum; RA_CristaTerminalis; lnferior_Mitral_Line; and LA_Septum.

At block 301 a 3D map of the heart of a patient is obtained. As mentioned above, the 3D map can be generated based on an image of the heart received from a suitable imaging system (220). If the left atrium is being examined, the 3D map contains at least a portion of the heart that includes the left atrium.

At block 303 an automatic map segmentation process is applied on the 3D map to identify multiple anatomical parts of one or more sub-compartment of the heart (e.g., left atrium). In some examples, the 3D map is received by automatic map segmentation module 221 configured to execute an automatic segmentation process to identify different parts of the heart in the 3D map. In some examples, a 3D image received by computer 210 is transformed into a 3D map using appropriate software, implemented for example as part of automatic map segmentation module 221.

In some examples the automatic map segmentation process includes applying a machine learning module on the 3D map configured for automatically segmenting the heart or atria (and possibly other sub-compartments of the heart as well) and providing data indicating different anatomical parts (or structures) of the heart or atria.

According to some examples, the automatic map segmentation process is the same process described in US Patent Application No. 18/207854, entitled "SYSTEMS AND METHODS FOR RECOMMENDING ABLATION LINES," filed on June 9, 2023 by the Applicant and published on December 14, 2023, having Publication No. US 2023-0397950. Application 18/207854 claims priority from US Provisional Patent Application No. 63/350,983, filed on June 10, 2022.

US Patent Application No. 18/207854, discloses an automatic map segmenter (22) configured to segment the anatomical map and output data identifying parts of the anatomy. The map segmenter utilizes a machine learning model (implementing a neural network and described in detail with reference to Fig. 3 in 18/207854) which receives the 3D map of the heart as input and provides a set of codes indicating the anatomical parts of the heart as output (e.g., anatomical parts of the atria). The machine learning model is trained using a training dataset that comprises a sufficient number of paroxysmal and persistent AF cases (e.g., 5000 or more cases) with anatomical maps of each case. A more detailed description of the training and execution of a machine learning model dedicated for segmenting the heart is disclosed in 18/207854.

In some examples, automatic map segmentation module **221** in Fig. 2 of the present case is configured in the same manner as Automatic Map Segmenter **22** disclosed in 18/207854. In other examples, it may retain certain properties of Automatic Map Segmenter 22, but not all. For example, the machine learning model described in 18/207854 is trained to provide both segmentation of parts of the heart or left atrium and ablation lines. In some examples, automatic map segmentation module **221** disclosed herein can be trained to provide segmentation of anatomical parts of the heart and/or left atrium, but not the ablation lines.

At block **305** the 3D map (now segmented) undergoes dimension reduction to obtain a corresponding 2-dimensional (2D) representation. In some examples these operations are carried out by applying Principal Component Analysis (PCA) that projects the 3D map to a 2D plane, as is well known in the art (e.g., by map dimension reduction module 223).

At block **307** different dimensions of the 2D representation of various anatomical parts of one or more sub-compartment of the heart are measured to obtain the respective dimension values. The measured dimensions include, but are not limited to, the height and width of the respective part.

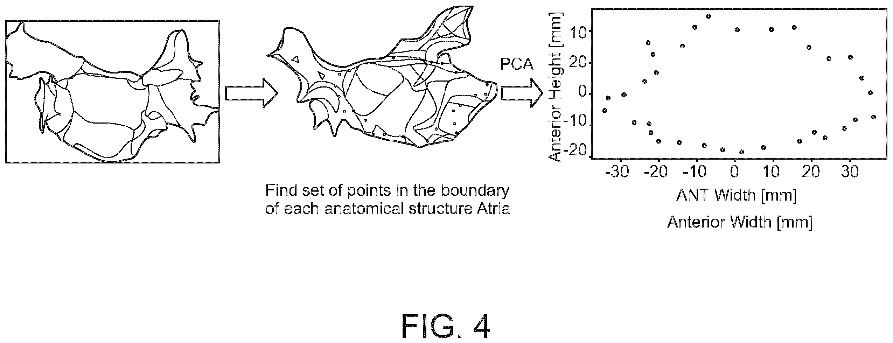

Fig. 4 shows on the left side (a) an image of the left atrium. In the middle (b) an anterior view of the atrium with anterior design line (indicated by dots) is shown, and its two-dimensional representation after PCA projection is shown on the right (c). In some examples, the anterior width (or height, when measured along the y-axis) is determined as the length in millimeters (mm) of the maximum point minus the minimum point along the x-axis (ory-axis). Mathematically, the width (or height) of an anatomical part (structure) is the length in mm of the maximum point minus the minimum point along the x-axis (or y-axis) of the points in the 2D plane. This calculation assumes that each one of the anatomical parts of the heart has constant characteristics, meaning that given two different dimensions of the atrium, one dimension is always greater than the second dimension. For example, it may be expected that the anterior length of an atrium is greater than its width.

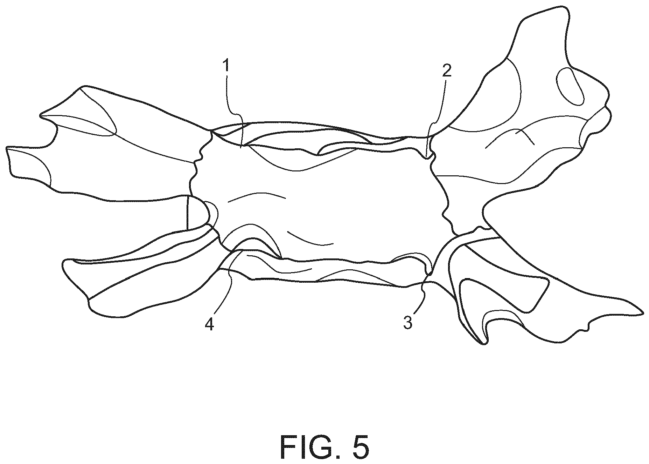

Additionally, according to some examples, posterior wall dimensions are calculated based on four anchor points, as shown in Fig. 5.
- Anchor point 1 - is the junction between LSPV, roof, and posterior walls.
- Anchor point 2 - is the junction between the RSPV, roof, and posterior walls.
- Anchor point 3 - is the junction between the RIPV, inferior, and posterior walls.
- Anchor point 4 - is the junction between the LIPV, inferior, and posterior walls.

Based on these anchor points, 8 types of left atrium dimension measurements are calculated:
a. Posterior Width 1 - the geodesic and Euclidian distance between anchor point 1 and anchor point 2.
b. Posterior Width 2 - the geodesic and Euclidian distance between anchor points 3 and 4.
c. Posterior Height 1 - the geodesic and Euclidian distance between anchor points 1 and 4.
d. Posterior Height 2 - the geodesic and Euclidian distance between anchor points 2 and 3.

At block 309 one or more parameters (also referred to herein as " anatomical prediction parameters") that are indicative of the likelihood of long-term success (or long-term failure) of a PVI only procedure administered to the AF patient, are determined. Examples of these parameters are listed in table 1 shown below in the "Examples" section.

In addition to the singular parameters ("singular anatomical prediction parameters" or "singular geometric parameters") determined based on the measured value of a single dimension of a single anatomical part of the heart (e.g., RLAT width, RLAT length, POST width, POST height, etc.) additional parameters are determined based on two or more singular parameters (also referred to herein as "complex anatomical prediction parameters" or "complex geometric parameters"). This can be accomplished, for example, by applying a mathematical operation (e.g., multiplication) between pairs or triplets of singular parameters to obtain the respective values. In some examples, the products of all pairs of singular values are calculated to obtain the additional parameters. In some examples, the products of all triples of singular values are calculated (in addition to or instead of the products of the pairs) to obtain the additional parameters. A list of complex anatomical parameters generated by multiplying pairs of singular parameters is shown below in table 2 the "Examples" section. Notably, the presently disclosed subject matter is not limited to pairs and triples of singular values, and in some examples complex anatomical prediction parameters can be calculated using a greater number of singular values.

The above calculations can be carried out, for example, by geometric parameters calculator 227 in system 210, which can be configured for determining singular geometric parameters and calculating complex geometric parameters. Notably, in case an anatomical prediction parameter is based on a single dimension, the dimension value determined in the previous stage can be used in a straightforward manner. If, however, an anatomical prediction parameter is based on more than one dimension, the corresponding calculation is executed.

In some examples, once one or more anatomical prediction parameters have been calculated, the parameters are compared to respective predefined values, e.g., threshold values set to differentiate between long-term success and long-term failure (block 311). For example, considering the width of right lateral wall (RLAT) of the left atrium, according to the results described below, a threshold value indicating long-term success can be equal to a value higher than 42.6 mm in order to achieve 75% sensitivity for detecting long-term failure. Based on the results of the comparison, either a long-term success or long-term failure of a PVI only procedure can be determined, and a corresponding recommendation can be generated, where in case of a predicted long-term success, PVI only treatment is recommended and otherwise, PVI plus is recommended. Notably, while Fig. 3a describes determination of a prediction with respect to PVI only, it is further contemplated to likewise predict long-term success of PVI plus using appropriate AF-related features and thresholds. In some examples, a group of different anatomical prediction parameters is used to increase robustness of the recommendation.

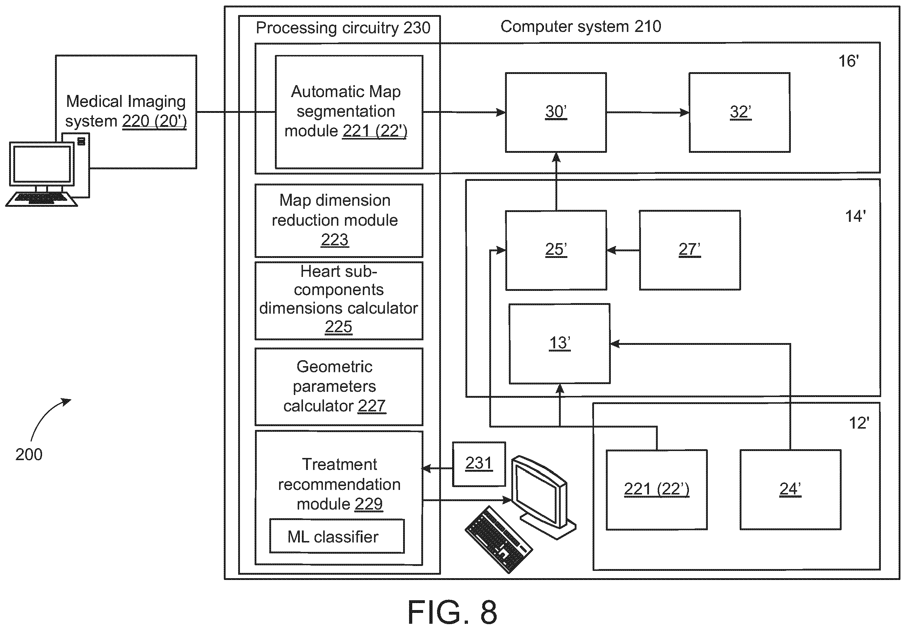

In some examples, the process continues by administering to the patient an appropriate treatment according to the recommendation (block 313). If long-term success of a PVI only procedure is predicted, PVI only treatment is administered to the patient, and if long-term failure of a PVI only procedure is predicted, a different treatment (e.g., PVI plus) is administered to the patient. Fig. 8 below shows an example of a computer system that combines the functionality of system 200 with additional functionalities, including the facilitation and guidance of an appropriate PVI treatment. In some examples, once a recommended treatment is determined the system can recommend suitable ablations lines, display the lines on respective map and guide the physician through the process.

The presently disclosed subject matter further contemplates a machine learning (ML) model dedicated for selecting an appropriate ablation approach (i.e., PVI or PVI plus) to AF patients. Fig. 3b is a flowchart showing operations carried out as part of a machine learning based analysis and treatment recommendation process, in accordance with some examples of the presently disclosed subject matter. The model is trained to associate between AF related features of an AF patient and a recommended treatment.

During a training phase a ML model is trained, using a training dataset that includes multiple features determined with respect to many different AF patients (herein below "AF related features"). According to one example, the model is trained to correlate between various combinations of AF related features and a respective probability of success of a PVI only procedure. In another example, the model is trained to correlate between various combinations of AF related features and a respective probability of success of PVI only procedure and/or PVI plus procedure.

A training dataset of AF related features of many different patients is obtained (block **301b).** The AF related features include at least one type of heart related features, which are attributes characterizing the heart of a patient, related to parameters of the heart. The AF related features may also include other AF related features which are not heart related.

One example of heart related features are anatomical prediction parameters (including both singular geometric parameters and complex geometric parameters, e.g., pairs and triples). Additional examples of heart related features that can be used during training and execution of the ML model include voltage related features and cycle length.

Voltage related AF features are based on voltage measurement values obtained from patients. The voltage measurements include, for example, measurements made at low-voltage zones (LVZ) areas, low voltage fractionation, and the like. Voltage measurement can be applied in various ways. For example, LVZ can be measured at each anatomical part (e.g., posterior wall, anterior wall, left lateral, right lateral, roof, and inferior wall). The voltage measurement can include, for example, one or more of, the absolute low voltage value measured over the entire area of the respective anatomical part, and a relative low voltage value representing the ratio between the measured low voltage and the voltage over the entire area of the anatomical part (e.g., posterior wall).

Additional voltage related features include global voltage related parameters such as different percentiles of voltage distribution (voltage patterns) in a part of the heart or its entirety, which may be based on different thresholds. For example, 25th percentile, 50th percentile, 90th percentile, and the like.

Voltage related features can be measured for example using the CARTO system by Biosense Webseter mentioned above. The FAM technique is designed to rapidly create a high-resolution 3D anatomical map of the heart chamber of interest, such as the left atrium. This is achieved by moving the mapping catheter along the endocardial surface while continuously recording its position and the corresponding electrical activity. FAM can be combined with voltage mapping for the identification of LVZs within the rapidly acquired anatomical map. Notably, in some examples global voltage can be used alone as a sole feature to determine PVI only or PVI plus.

As mentioned above, certain voltage measurements can be applied on multiple individual anatomical parts of the heart and/or atrium, thus obtaining a plurality of different voltage related features from a single patient.

Cycle length refers to any type of measurement of the time interval between detected electrical activations. For example, activations measured on a CS (Coronary Sinus) catheter, HRA (High Right Atrium) catheter, locally placed catheter, or other.

AF related features which are not heart related include, for example, patients' demographics, including various details of personal information characterizing the patients, such as gender, age, place of residence, and so forth.

It is noted that while various examples of types of AF related features are mentioned above, in different examples only a subset of these features are used during training and execution of the machine learning model. It is further noted that the examples of AF features disclosed above should not be construed as limiting, and other types of AF related features are also contemplated to be within the scope of this disclosure.

To obtain the AF related features for training the model, data from multiple AF patients (e.g., from thousands to tens of thousands of patients or more), is collected and the respective parameters are calculated from the data of each patient and used as features in the training dataset. The AF related features can be obtained for example during Atrial Fibrillation ablation procedures. If available, a database of AF patients that includes parameters obtained during catheterization and/or ablation can be used.

The training dataset is used for training a machine learning model (block 303b). Different types of classifiers can be generated. These classifiers include for example, a classifier providing data indicative of probability of long-term success of a Pulmonary Vein Isolation (PVI) only procedure (generated for example using a training dataset comprising data obtained from AF patients that were treated by a PVI only procedure), a classifier providing data indicative of probability of long-term success of a PVI plus procedure (generated for example using a training dataset comprising data obtained from AF patients that were treated by a PVI plus procedure), and a classifier providing data indicative of probability of long-term success of both PVI only and PVI plus (generated for example using a training dataset comprising data obtained from a collection of AF patients that were treated by either PVI only or PVI plus procedure).

According to some examples, the machine learning model is implemented as a supervised model, where patients (data points) in the training dataset are tagged according to their respective observed long-term success or long-term failure of a PVI only procedure, representing the ground truth. This approach requires obtaining valid information from a sufficiently large cohort of AF patients indicating observed long-term success or failure of a PVI only procedure. In other examples, data points in the training dataset are tagged according to their respective observed long-term success or long-term failure of a PVI only procedure and/or PVI plus procedure.

Training can be executed for example by computer 210 or by another computer remotely connected to computer 210 (e.g., by ML classifier training module **231** which can be configured to receive the training dataset and train the model). The trained ML model can be stored in a computer data-repository (235) and made available to be used for selecting an appropriate ablation approach to AF patients (block **305b).**

The above approach can be implemented in case such a cohort of AF patients is indeed available. However, it also creates a technical challenge which precludes the implementation of such a machine learning model if this information is unavailable. To overcome this technical shortcoming, it is suggested herein to use an alternative approach where the machine learning model is trained based on the decisions made by physicians that examined the AF patient and recommended on an ablation approach (and possibly also administer the treatment), rather than the actual observed outcome of the treatment, and thus overcome the technical setback resulting from insufficient training data. This alternative approach is based on the assumption that aligns with "Wisdom of the crowd" (WoC) principle. According to this principle, in large scale (involving for example, hundreds of thousands or millions of unrelated cases or more), the majority of individuals make the correct decision.

The use of physician recommendations in training the ML model addresses the technical problem of data scarcity related to the actual outcomes of AF treatments, and leverages expert clinical judgment as a reliable surrogate for outcome data, thereby facilitating the generation of a robust and effective ML model, despite the limited availability of outcome data.

In the present context, "individuals" are physicians, and the "decision" at hand pertains to a recommendation whether to administer PVI only, or the PVI plus procedure, and, in some examples, also which type of PVI plus procedure. Thus, although here the ground truth is a recorded medical recommendation, and not an observed success or failure, it is considered a good predictor of success or failure, which can be used for selecting the appropriate ablation approach.

According to some examples, a patients' database that complies with certain requirements is used for this purpose. These requirements include, for example:
Size: The database is large enough to provide a statistically viable analysis, storing information on many different AF patients, such as thousands or tens of thousands of AF patients and many different physicians.
Diversity: The database includes many AF patients with a variety of paroxysmal and PS-AF cases, characterized by different features. These features may include varying atria shapes, different voltage patterns (i.e., the distribution of low voltage over different areas of the atria), and different ages and genders. The diversity of the database can be determined using known statistical methods.
Independence: The database contains many patients with similar AF characteristics who were treated independently by different doctors at different medical institutions. Similarity between different AF patients in the database can be determined by applying a clustering mechanism on the data, such as a Gaussian Mixture Model (GMM), spectral clustering, K-means, etc.

According to one approach, training can be based on the actual opinion of a cohort of doctors presented with different AF patients to obtain the opinion of many doctors regarding each case. This approach can use either binary or multi-class classification. This approach can be adopted if the data includes recommendations made by many physicians with respect to specific "real" patients.

However, since such data is many times unavailable a different approach that overcomes the deficiency in the data is suggested. As the database is characterized by high diversity in the recorded cases on one hand, and similarity between cases on the other, it in fact includes groups (or classes) of patients with similar AF characteristics, where each group can be considered to correspond to a certain AF patient ("virtual patient"). The inventors found that the independent medical opinions made by the many different physicians with respect to similar cases, all assigned to the same group, can be viewed as if made with respect to the same AF patient. Therefore, for the purpose of training the machine learning model, the data can be viewed as if many different physicians gave a medical opinion to the same patient. As mentioned above, in large scale it can be assumed that a majority vote of the medical opinion made by physicians with respect to each group of patients is likely to be the option of choice for a specific patient.

According to the WoC approach, the machine learning model can be implemented for example as a supervised model, where patients in the training dataset are tagged according to the respective medical opinions made by the different physicians recommending PVI only or PVI plus treatment, representing the ground truth.

Whether using the first approach, where probability of success is based on observed long term success of PVI only or PVI plus treatment, or the second approach (WoC) where a recommendation of treatment is based on treatment choice made by physicians, the machine learning classifier is trained, using the training dataset, to correlate between various combinations of AF related features and a respective prediction. The training dataset can be used for training a suitable classifier machine learning model, such as a support vector machine (SVM), random forest, or logistic regression. If the training dataset is sufficiently large, a deep neural network can be used for classification, such as a convolutional network with dense layers.

The ML model can be trained to provide one or more training outputs. According to one example, the ML model can be trained to provide binary classification providing a recommendation indicating which approach is more likely to be effective in treating AF for a particular patient, PVI only, or a PVI plus.

When implementing the ML model according to the WoC approach, according to one example each patient is tagged individually according to the actual treatment that was administered to the patient or according to the treatment that was recommended. For example, 0 for PVI only, and 1 for PVI plus.

In case a multi-class classifier is trained, comprising a PVI only class, and several PVI plus classes, each corresponding to a different type of additional ablation line (e.g., additional ablation line in anatomical parts of the heart other than pulmonary veins of the left atrium) tagging can be likewise done according to individual treatment and/or recommendation of each patient.

According to the example where patients in the training dataset are clustered into groups according to similarity between their respective AF features (using a clustering mechanism as mentioned above), each group representing a type of patient ("virtual patient"), tagging can be done according to the collective distribution of decisions made with respective to different virtual patients by all physicians.

Considering a simplified example, assume there are two classes of patients (virtual patients) each corresponding to patients with common AF related features, and there are two physicians, with the following recommendation distribution:

| | Physician A (PVI/PVI+) | Physician B (PVI/PVI+) | Collective tagging approach |
|---|---|---|---|
| Virtual patient a | 20/80 | 10/90 | 15/85 |
| Virtual patient b | 50/50 | 40/60 | 45/55 |

According to the collective tagging approach, the tagging of patients is determined according to the combined information from all physicians (e.g., by calculating a weighted average). This collective tagging approach reflects a consensus or averaged decision, leveraging the combined expertise of the physicians for more balanced and robust tagging of patient clusters.

According to some examples, the machine learning model is implemented as an ensemble of supervised machine learning classifiers, where each classifier in the ensemble represents a virtual physician providing a respective recommendation. In some instances, the classifiers in the ensemble may all be of the same type of ML model, while in other instances, they may be of different types of models (e.g., Random Forest, XGBoost, etc.). The training set can be divided (e.g., randomly) into groups, where each classifier is trained using a subset of the data points (patients) in a group. During inference each classifier provides a respective recommendation, and the final recommendation is determined based on a majority vote.

If the training dataset is sufficiently large and contains enough recommendations made by different physicians, labs, or other medical institutions (occupying multiple physicians), it can be divided into groups according to the physicians or labs. This allows the creation of individual ML models, each representing a "virtual physician", associated with a real-life physician or lab.

Fig. 6 is a flowchart showing an example of training of multiple models according to the WoC approach. Notably, the same type of ensemble can be used using a cohort of patients with recorded observed success or failure of PVI treatment if such data is available.

At block 601 a training dataset of AF patients is generated; each patient is associated with a respective AF related feature and PVI treatment recommendation. The patients are divided into sub-groups, where in some examples each sub-group comprises patients with similar AF features (block 603). As explained above, in some examples, each sub-group comprises patients who have been recommended AF therapy by a certain physician or lab.

The patients in each sub-group are tagged according to the observed recommendations made by the respective physician or lab (block 605). An individual or collective tagging approach can be implemented.

These sub-groups are then used to train a respective classifier for each physician-related or lab-related sub-group of patients (block 607). This results in multiple classifiers, each corresponding to the decision-making process of a specific physician or lab, effectively acting as a virtual physician providing predicted recommendations for the patients.

This approach allows for personalized and context-specific predictions, as each model is fine-tuned based on the medical preferences and opinions of individual physicians or labs. By leveraging these specialized models, the system can enhance the accuracy and relevance of its recommendations, ultimately leading to better patient outcomes and more efficient healthcare delivery, while overcoming the technological challenge mentioned above resulting from the scarcity of data on completed AF treatments.

Furthermore, in both approaches (observed success and WoC) an additional output of the model includes data indicating the significance of different AF related features in predicting the final ML output related to the recommended treatment. In the context of ML models, individual features, or any combination of AF related features (e.g., a particular singular geometric parameter, a particular complex geometric parameter, a combination of two or more parameters of any type, voltage AF related features, and so on), could be crucial for determining the recommended treatment. The ML model disclosed herein can be used for identifying the most significant feature or features in providing the recommendation. Providing this information to a physician would help the physician to rationalize the recommendation made by the model, thereby further assisting the physician when making a final decision how to treat the patient. This can be achieved by applying a features importance analysis. For example, in case a Decision tree or Random Forest model is used, a Gini index can be used to quantify features' importance.

Reverting to Fig. 3b, in the execution phase (inference), the trained ML model can be applied to determine a recommended treatment for a particular AF patient. Notably, although the training phase and execution phase are illustrated in sequence, as is well known in the art, the two phases can be executed asynchronously.

During execution, the AF related features obtained with respect to one or more AF patients are applied to the ML model to obtain a respective recommendation for each patient, as to whether administer to the patient PVI only or PVI plus treatment.

Execution of the ML model can be done as part of the process described above with reference to Fig. 3a. Assuming a certain AF patient is a candidate for the ablation approach, and the physician wishes to receive a recommendation which ablation approach is more suitable for the patient, the physician can use system 220 for applying the ML model for this purpose.

To this end, AF related features of patients are obtained (307b). AF related features (e.g., heart related features) can be obtained by applying appropriate procedures on the patient dedicated for measuring relevant values, and performing further processing on the measured values, if necessary. For example, anatomical prediction parameters can be obtained by applying the procedure as described with reference to blocks **301** to 309 in Fig. 3.

Multiple anatomical prediction parameters, both singular and complex, can be calculated (e.g., by geometric parameters calculator 227) to provide multiple AF related features, which are recorded as part of an input dataset of AF related features for the ML model.

Other types of AF related features can be obtained by applying additional procedures on the patient or the relevant data. For example, various types of voltage related features can be obtained by performing different voltage measurements on the patient's heart and adding the obtained voltage values to the input dataset. Voltage and cycle length measurements can be performed using appropriate medical instruments for example, using an intracardiac electrocardiogram operatively connected to system 200. A patient's demographics can be obtained, for example, from the patient's medical records.

The ML model is applied to the input dataset of AF related features obtained from the patient and the ML model output is obtained (block 309b). The ML model output is provided including a recommendation indicating whether to administer PVI or PVI plus to the patient (block 311b). As further explained above, in some examples the ML model provides additional information indicating, for a PVI plus recommendation, which additional treatment, or treatments, are the recommended treatment for the patient. As explained above, in some examples the ML model also provides information indicating which of the input features had the greatest contribution to the model's output. In some examples, the process continues by administering to the patient an appropriate treatment according to the recommendation (block 313b).

Fig. 7 schematically illustrates inference using an ensemble of "virtual physicians" classifiers, where an ML model, which includes an ensemble of classifiers, is used for generating a treatment recommendation to an AF patient. Each one of the machine learning models in the ensemble is applied to the AF related features of the patient to obtain respective outputs. Once the outputs from all classifiers in the model are obtained, a majority vote is applied, and an appropriate recommendation is determined accordingly.

In some examples, operations described above with respect to blocks **309b** and **311b** in Fig. **3b** are carried out by treatment recommendation module **229.** Treatment recommendation module **229** can include, for example, ML classifier module configured to apply ML classifier (or ensemble of classifiers) to the AF related features of the patient, as described above.

It is further noted that US patent application 18/207854, describes (e.g., with reference to Figures 2 and 3) a system for rendering recommended ablation line(s) on an anatomy (herein below "rendering system"). In some examples, system **200** disclosed herein can be incorporated as part of the rendering system, providing the rendering system with the capability to generate a recommendation as to whether PVI only treatment is suitable for a patient. In other examples, system **200** can be implemented as a stand-alone system separated from the rendering system described in 18/207854. Regardless, system **200** and the rendering system may, in some examples, share certain components. For example, medical imaging system **20** and automatic map segmenter **22** shown in Fig. 2 of 18/207854 correspond to medical imaging system **220** and automatic map segmentation module **221** in Fig. 2 of the present application, respectively.

Fig. 8 is schematic illustration of a computer system that combines the functionality of system **200** disclosed herein and deep learning system **10** disclosed in 18/207854 and 63/350,983. In Fig. 8 reference numerals with a prime sign identify elements disclosed in 18/207854 and 63/350,983, and reference numerals without a prime sign identify the corresponding elements disclosed in the present application. As explained in detail in 18/207854 the system in Fig. 8 includes three sections, an ablation line trainer 12 which builds a trained deep learning unit for an ablation line proposer 13, an ablation line recommender 14 which generates ablation line recommendations using ablation line proposer 13, and an ablation line guider 16 which provides ablation line guidance during a procedure. Ablation line trainer 12 may receive the anatomical map of the heart onto which the recommended ablation line(s) may be rendered and train a machine-learning model utilized by ablation line proposer 13 to output proposed ablation lines according to heart anatomy of a patient.

In some examples, ablation line recommender 14 receives data indicating a recommended ablation approach from treatment recommendation module 229 and uses this information when recommending the ablation lines, where the recommended ablation lines are selected to suite the recommended ablation approach. For example, in case of PVI only treatment, the recommended ablations are suited to PVI only treatment. Ablation line guider 16 provides ablation line guidance to the physician during a procedure. It may receive an anatomical map of the heart from medical imaging system 20 during the procedure and may comprise automatic map segmenter 22 to indicate the various elements of the heart during the procedure.

### Examples

Table 1 displays results of a statistical analysis performed by the inventors supporting the correlation between certain geometrical properties of the (left) atria and prediction of long-term success or long-term failure of the PVI only procedure. The data includes 95 cases of AF patients undergoing the PVI only procedure. Long-term failure of the PVI only procedure was defined according to a composite of 3 outcomes: 1) repeated ablation; 2) hospitalization for direct current cardioversion; or 3) AF related hospitalization. Patients who encountered any one of the above three conditions, within up to 12 months following PVI only treatments, were classified to the long-term failure subset. 16 subjects out of the 95 exhibited long-term failure according to the above definition.

Table 1 shows basic atria parameters (singular parameters) and their relation to long-term success prediction. Table 1 (as well as Table 2) shows, for each parameter, the P-value indicating the difference between the long-term success values and the long-term failure values. Posterior height and posterior width based on geodesic distances, for example, are significant in differentiating long-term success from long-term failure for Persistent Atrial Fibrillation (PS-AF) subjects based on PVI only.

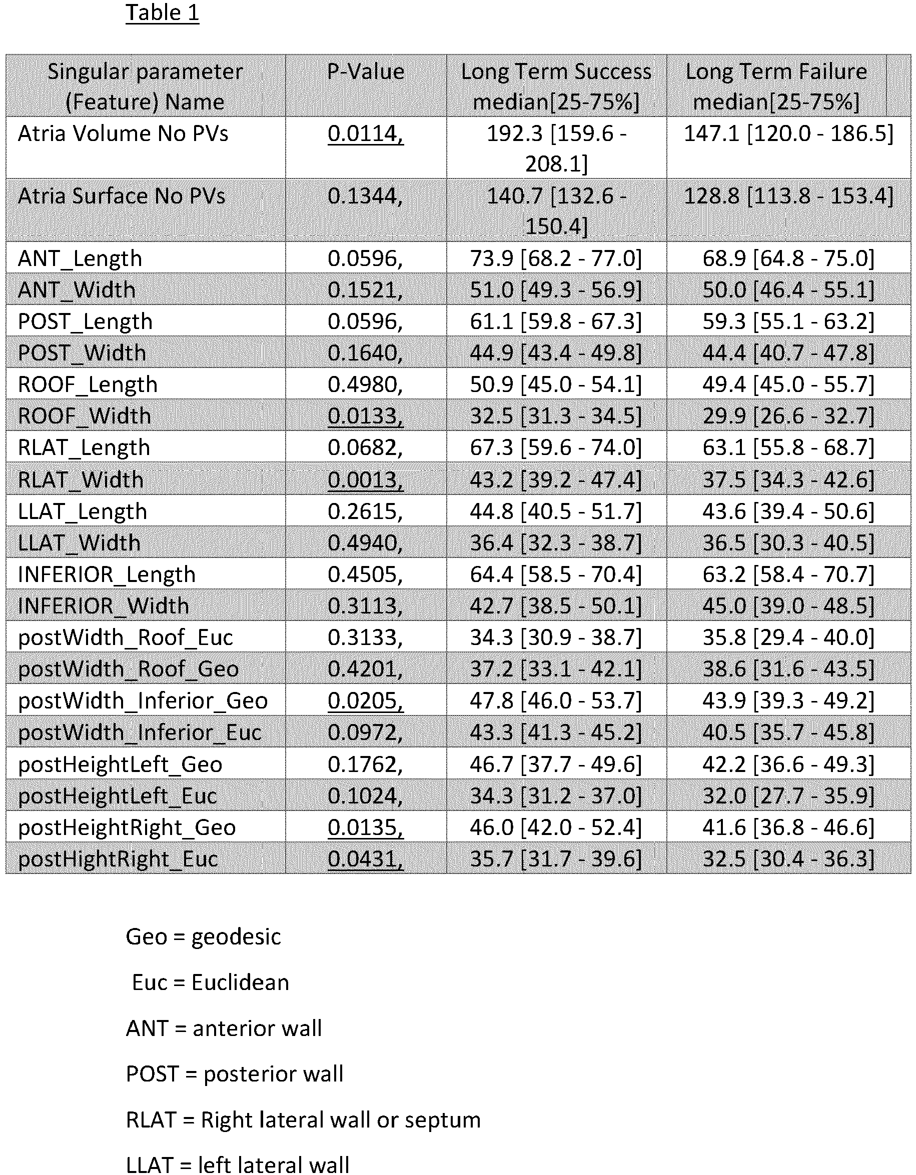

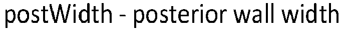

In the left column of the table different parameters (features) of dilation of the left atrium are listed. Each parameter corresponds to a certain anatomical part of the heart, and is calculated based on one or more dimensions of the anatomical part. The correlation between the value of the different parameters (obtained before treatment) and long-term success or failure of the PVI only procedure was examined. The listed parameters include: anterior (ANT) length and width, posterior wall of the left atrium (POST) length and width, roof length and width, right lateral wall (RLAT) length and width, inferior length and width, LLAT width and length.

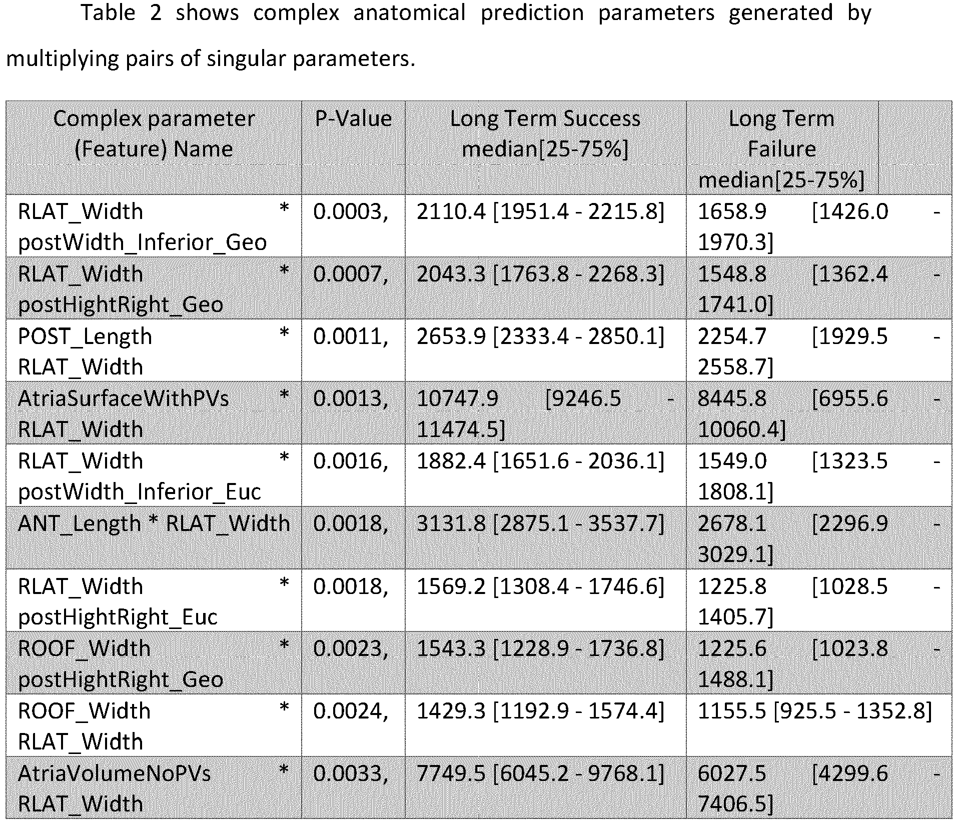

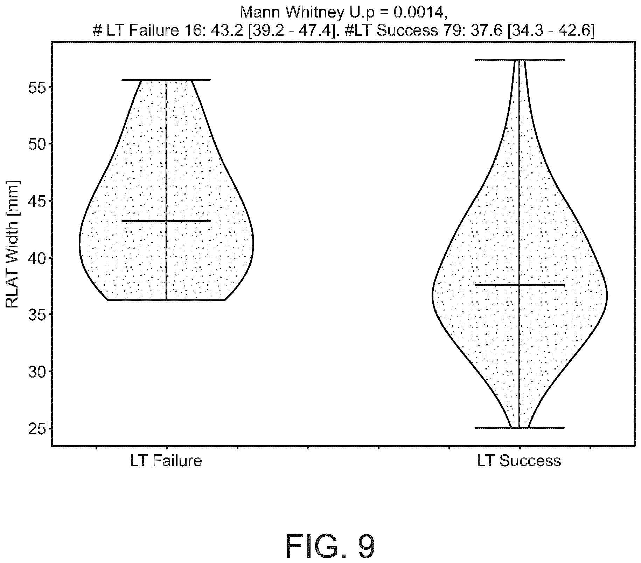

Based on this study, the most prominent singular parameter was the right lateral wall width which increased significantly (p-value <0.005) from 37.5 [34.3 - 42.6] mm for patients who succeeded in the PVI procedure to 43.2 [39.2 - 47.4] mm for patients who failed in the procedure. This is further demonstrated in Fig. 9 that shows a comparison between RLAT width in patients that exhibited long-term (LT) failure and patients that exhibited long-term success of the PVI only procedure.

The inventors also found an increase in height and width of the posterior wall, and an increased width of the roof for subjects who failed the PVI procedure. By multiplying each pair of statistically significant parameters, the inventors obtained a new set of parameters that were more significant in distinguishing between long-term failure and long-term success.

### Experiment Validating WoC-based ML Model

An experiment was conducted to validate the efficacy of an ML model trained using the WoC approach. A cohort of actual AF patients, who had undergone either PVI-only or PVI-plus treatments, was assembled along with their outcomes, specifically the period of AF and arrhythmia-free period. The ML model was then applied to the AF related features of this cohort of patients, and the respective recommendations (PVI or PVI-plus) were obtained from the model. The patients were divided into four groups, based on the ML model's recommendations and the actual treatments they received:
1. Patients recommended PVI by the ML model who received PVI.
2. Patients recommended PVI by the ML model who received PVI-plus.
3. Patients recommended PVI-plus by the ML model who received PVI-plus.
4. Patients recommended PVI-plus by the ML model who received PVI.

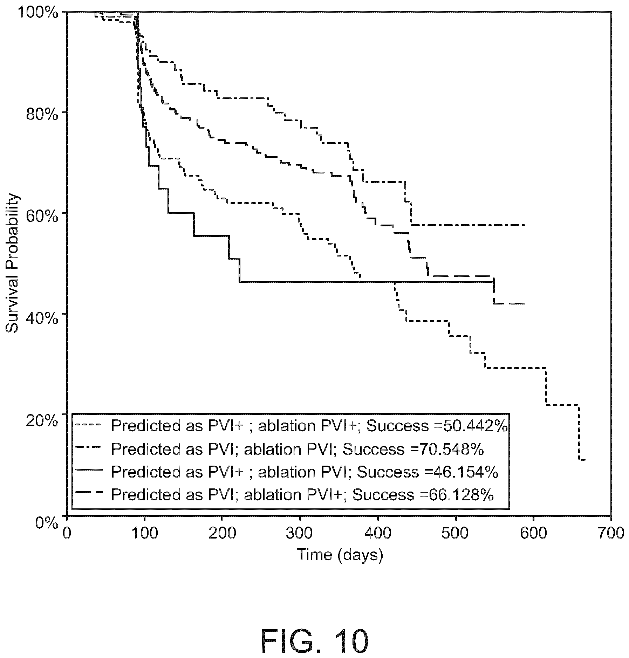

Fig. 10 presents a graph illustrating the survival probability of AF patients over time, defined as the period free from AF and arrhythmia. Table 3 below details the probabilities of achieving a 1-year AF and arrhythmia-free period for each group of patients, based on the observed outcomes.

| **Group** | **Probability 1 year freedom from AF** |
|---|---|
| PVI recommended, PVI performed | 70.5% |
| PVI recommended, PVI+ performed | 66.1% |
| PVI+ recommended, PVI+ performed | 50.4% |
| PVI+ recommended, PVI performed | 46.1% |

The analysis reveals that alignment between the ML model's recommendations and the actual treatments administered correlates with improved outcomes in terms of 1-year freedom from AF and arrhythmia. Specifically:
- Patients for whom the ML model recommended PVI and who received PVI demonstrated the highest 1-year AF and arrhythmia-free probability (70.5%).
- Patients recommended PVI by the model but who received PVI-plus had a slightly lower 1-year AF and arrhythmia-free probability (66.1%).
- Patients recommended and treated with PVI-plus showed a 1-year AF and arrhythmia-free probability of 50.4%.
- Patients recommended PVI-plus but who received PVI only had the lowest 1-year AF and arrhythmia-free probability (46.1%).

Comparison of Results:
First Group vs. Second Group: Patients who were recommended PVI and received PVI (first group) had a higher 1-year AF and arrhythmia-free probability (70.5%) compared to those who were recommended PVI but received PVI-plus (second group) with a 1-year AF and arrhythmia-free probability of 66.1%. This indicates that following the ML model's recommendation for PVI results in better outcomes than deviating from the recommendation and performing PVI-plus.
**Third Group** vs. **Fourth** Group: Patients who were recommended PVI-plus and received PVI-plus (third group) had a higher 1-year AF and arrhythmia-free probability (50.4%) compared to those who were recommended PVI-plus but received PVI (fourth group) with a 1-year AF and arrhythmia-free probability of 46.1%. This suggests that adhering to the ML model's recommendation for PVI-plus provides better outcomes than performing PVI instead.

These results, as shown in the survival probability graph in Fig. 10 and detailed in Table 3, suggest that the ML model's recommendations are strongly correlated with treatment success. Adherence to the model's guidance can enhance clinical outcomes, underscoring the critical importance of following the ML model's recommendations to achieve optimal clinical results.

The various illustrative logical blocks, modules, and algorithm steps described in connection with the examples disclosed herein can be implemented as electronic hardware, computer software, or combinations of both. To clearly illustrate this interchangeability of hardware and software, various illustrative components, blocks, modules, and steps have been described above generally in terms of their functionality. Whether such functionality is implemented as hardware or software depends upon the particular application and design constraints imposed on the overall system. The described functionality can be implemented in varying ways for each particular application, but such implementation decisions should not be interpreted as causing any departure from the scope of the disclosure.

It will also be understood that a system according to the present disclosure may be, at least partly, implemented on a suitably programmed computer. Likewise, the present disclosure contemplates a computer program being readable by a computer for executing the method of the present disclosure. The present disclosure further contemplates a non-transitory computer-readable memory tangibly embodying a program of instructions executable by the computer for executing the method of the present disclosure.

Those skilled in the art will readily appreciate that various modifications and changes can be applied to the examples of the invention as hereinbefore described without departing from its scope, defined in and by the appended claims.

## Claims

1. A computer-implemented method of selecting an Atrial Fibrillation (AF) treatment, the method comprising:
obtaining a plurality of AF related features of a patient; the AF related features of the patient include heart-related features characterizing various heart-related attributes of the patient;
the heart-related features include at least one of: one or more anatomical prediction parameters, each anatomical prediction parameter being calculated based on geometrical data collected from a particular anatomical part of the heart; and one or more voltage-related features;
applying a machine learning (ML) model to the plurality of AF related features of the patient; wherein the ML model has been trained using a training dataset that includes multiple data points, each data point corresponding to an AF patient associated with AF related features and a corresponding recommendation made by a physician to administer to the patient an AF treatment selected from a group including at least Pulmonary Vein Isolation (PVI) only and PVI plus treatments, thus training the ML model to provide, during inference, data indicating a recommended AF treatment to a given patient according to the AF related features of the given patient;
generating an ML model output indicative of a recommended AF treatment for the patient, selected from a Pulmonary Vein Isolation (PVI) only procedure, and a PVI plus procedure; and
providing data indicative of the recommended AF treatment to a healthcare provider.

2. The method of claim 1, wherein the heart related features include anatomical prediction parameters, the method further comprising obtaining anatomical prediction parameters of the patient:
performing a procedure on the patient for obtaining a 3D anatomical map of at least one sub-compartment of a heart of the patient, selected from a group comprising: left atrium, right atrium, and left ventricle;
segmenting the anatomical (3D) map to thereby obtain multiple anatomical parts of the at least one sub-compartment;
for at least part of the multiple anatomical parts, determining at least one respective dimension;
determining anatomical prediction parameters, wherein each anatomical prediction parameter is determined based on one or more dimensions of an anatomical part of the at least one sub-compartment of the heart of the patient.

3. The method of claim 2 comprising: determining at least one anatomical prediction parameter by applying a mathematical operation between different dimensions of a respective anatomical part of the at least one sub-compartment.

4. The method of claim 1, wherein the AF related features further include one or more of: cycle length, medical history, and patients' demographics.

5. The method of claim 1, wherein the ML model is an ensemble of classifiers each classifier is trained on a subset of the data points and configured to provide a respective output indicative of a recommended AF treatment for the patient; and wherein a final recommendation is determined based on a majority vote.

6. The method of claim 5, wherein each subset of data points includes patients that were recommended by a particular physician, lab or medical institution, thus representing a virtual physician.

7. The method of claim 1, wherein the machine learning model output is further indicative of a respective significance of different AF related features in determining the recommended AF treatment for the patient.

8. The method of claim 1, wherein during training, clustering is applied on the multiple data points to thereby assign subset of patients **characterized by** similar AF related features to respective groups, each group representing a patient type, and tagging the patients in the group according to distribution of recommendations made by physicians in each group.

9. The method of claim 1, wherein the ML output that includes a recommendation to apply a PVI plus procedure on the patient also includes information indicative of a particular type of recommended PVI plus procedure.

10. A computer program product comprising a computer readable storage medium retaining a program of instructions, which, when read by a computer processor, causes the computer processor to perform a method according to any one of claims 1 to 9.

11. A computer system comprising at least one processing circuitry comprising one or more computer processors configured to execute operations according to any one of claims 1 to 9.
